# EUROPÄISCHE PATENTSCHRIFT

(11) **EP 2 712 643 B1**
(45) Veröffentlichungstag und Bekanntmachung des Hinweises auf die Patenterteilung: **21.03.2018**
(21) Anmeldenummer: 12006713.7
(22) Anmeldetag: 26.09.2012
(51) Int. Cl.: A61M 15/00

(54) **Inhalator**
Inhalator
Inhalateur

(43) Veröffentlichungstag der Anmeldung: 02.04.2014
(73) Patentinhaber: BOEHRINGER INGELHEIM INTERNATIONAL GMBH, 55216 Ingelheim (DE)
(72) Erfinder: Jung, Andree, 55216 Ingelheim am Rhein (DE); Dunne, Stephen Terence, Stowmarket, IP14 2 ED (GB)
(74) Vertreter: Von Rohr Patentanwälte Partnerschaft mbB

(56) Entgegenhaltungen:
- EP-A2- 1 707 232
- WO-A2-2008/034504
- US-A1- 2009 095 294

## Beschreibung

Die vorliegende Erfindung betrifft einen Inhalator gemäß dem Oberbegriff des Anspruchs 1.

Die vorliegende Erfindung betrifft insbesondere einen Inhalator zur Ausgabe bzw. Inhalation einer vorzugsweise pulverförmigen Formulierung, also einen Pulverinhalator. Jedoch kann die Formulierung grundsätzlich auch in flüssiger Phase, als Dispersion oder in sonstiger fluidisierbarer Form vorliegen.

Bei der Formulierung handelt es sich insbesondere um ein therapeutisches Mittel bzw. Arzneimittel. Insbesondere enthält die Formulierung dementsprechend mindestens einen Wirkstoff oder besteht daraus. Die Formulierung dient also insbesondere der medizinischen Behandlung oder sonstigen therapeutischen Zwecken.

Bei der vorliegenden Erfindung ist die Formulierung in oder von einem Träger aufgenommen, insbesondere vordosiert in einzelnen Dosen.

Die WO 2008/034504 A2, die den Ausgangspunkt der vorliegenden Erfindung bildet, offenbart einen Inhalator zur Inhalation einer Formulierung von einem Träger, wobei zum oder beim Austrag und/oder Dispergieren der Formulierung zumindest ein Teil des Trägers durch einen Luftstrom in Schwingung versetzt wird. Der Träger ist insbesondere flach, blisterartig und/oder folienartig ausgebildet. Fig. 3 der WO 2008/034504 A2 zeigt eine Ausführungsform, bei der der Träger direkt vom Luftstrom bewegt bzw. in Schwingung versetzt wird. Problematisch ist, dass der Träger quer zu seiner Flachseite angeströmt wird und frei flattert.

Der vorliegenden Erfindung liegt die Aufgabe zugrunde, einen verbesserten Inhalator anzugeben, so dass auf einfache Weise eine effektive Ausbringung und insbesondere Zerstäubung einer insbesondere pulverförmigen Formulierung ermöglicht wird und/oder ein einfacher und/oder kostengünstiger Aufbau, insbesondere als Einweginhalator, ermöglicht wird.

Die obige Aufgabe wird durch einen Inhalator gemäß Anspruch 1 gelöst. Vorteilhafte Weiterbildungen sind Gegenstand der Unteransprüche.

Vorschlagsgemäß weist der Inhalator einen durch einen Luftstrom insbesondere direkt bewegbaren bzw. in Schwingung versetzbaren Träger auf. Besonders bevorzugt weist der Träger eine die Formulierung enthaltende Aufnahme auf. Alternativ kann der Träger auch mehrere, vorzugsweise zwei Aufnahmen, insbesondere mit unterschiedlichen Formulierungen, aufweisen.

Der Träger ist mittels eines Federabschnitts beweglich gehalten. Der Träger ist vorzugsweise unmittelbar durch einen Luftstrom zum Austrag und/oder Dispergieren der Formulierung(en) bewegbar, insbesondere in Schwingung versetzbar.

Der Federabschnitt weist zwei parallel verlaufende Federstege und eine Durchbrechung, insbesondere einen Längsschlitz, auf. Dies ist einer definierten Bewegung, Amplitude und/oder Frequenz des Trägers zuträglich.

Ein bevorzugter Aspekt der vorliegenden Erfindung liegt darin, dass der Träger definiert in Schwingung versetzbar ist bzw. schwingt, insbesondere mit einer definierten Bewegung, mit einer definierten Amplitude und/oder mit einer definierten Frequenz. Dies ist einer verbesserten und definierten Ausgabe und Zerstäubung bzw. Dispergierung zuträglich.

Vorzugsweise ist unter einer "definierten Bewegung" eine über nur gewisse bzw. bestimmte Freiheitsgrade aufweisende Bewegung zu verstehen. Besonders bevorzugt ist der Träger zumindest im Wesentlichen nur um eine bestimmte bzw. von dem Federabschnitt gebildete Achse bzw. rotatorisch bzw. nur in Form einer Schwenkbewegung bewegbar, insbesondere wobei die Bewegungsbahn des Trägers in einer Ebene liegt und eine Haupterstreckungsebene des flachen Trägers zumindest im Wesentlichen immer senkrecht zu dieser Bewegungsebene verläuft.

Unter dem Begriff "definierte Amplitude" ist vorzugsweise eine Begrenzung der Bewegung bzw. Amplitude des Trägers zu verstehen, wobei hierzu bedarfsweise auch eine nur einseitige, also nur in einer Bewegungsrichtung wirkende Begrenzung, beispielsweise durch einen Anschlag, vorgesehen sein kann.

Unter dem Begriff "definierte Frequenz" ist vorzugsweise eine beschränkte Frequenz oder ein beschränkter Frequenzbereich zu verstehen, mit dem der Träger bei Benutzung schwingt. Da bei der Benutzung, insbesondere wenn der Luftstrom durch das Einatmen eines Benutzers erzeugt wird, unterschiedliche Luftströme auftreten können, ist der bestimmte Frequenzbereich der Schwingung vorzugsweise in Abhängigkeit von einem bestimmten Volumenstrom oder einem bestimmten Volumenstrombereich zu verstehen.

Der Träger bzw. Federabschnitt ist vorzugsweise nur an einer Seite fixiert oder gehalten, insbesondere eingespannt oder befestigt. Der Träger bzw. Federabschnitt endet an der anderen Seite frei bzw. so, dass die andere Seite (frei) schwingen kann.

Ein anderer Aspekt der vorliegenden Erfindung liegt darin, dass der Federabschnitt den Träger quer zum Luftstrom vorzugsweise beweglich, aber in Richtung des Luftstroms zumindest im Wesentlichen torsionssteif hält. Dies ist einer definierten Bewegung, Amplitude und/oder Frequenz des Träges zuträglich.

Gemäß einem anderen Aspekt der vorliegenden Erfindung ist der Träger zumindest im Wesentlichen ausschließlich von einem freien bzw. dem Federabschnitt entgegen gesetzten Ende her vom Luftstrom anströmbar. Dies ist einem optimalen Austrag der Formulierung zuträglich, insbesondere da unnötige Verwirbelungen im Inhalator vermieden oder minimiert werden können. Alternativ oder zusätzlich ist dies einer definierten Schwingung des Trägers zuträglich.

Gemäß einem weiteren Aspekt der vorliegenden Erfindung ist dem Träger vorzugsweise ein schrägstehender, vom Luftstrom anströmbarer Flügel zugeordnet. Besonders bevorzugt ist der Flügel an einem freien Ende des Trägers und/oder benachbart zu einem Lufteinlass des Inhalators angeordnet. Dies gestattet einen kompakten Aufbau und ein definiertes Anströmen des Trägers und/oder ein effizientes Einwirken auf den Träger, um diesen in Schwingung zu versetzen.

Gemäß einem weiteren Aspekt der vorliegenden Erfindung ist der Träger oder eine Abdeckung des Trägers vorzugsweise zumindest partiell durch eine Abdeckeinrichtung abgedeckt, die zum Öffnen des Trägers bzw. der Abdeckung für eine anschließende Ausgabe der Formulierung manuell öffenbar oder entfembar ist. Besonders bevorzugt erfolgt das Öffnen oder Entfernen der Abdeckeinrichtung durch Ziehen an einem Griffelement oder Abdeckelement der Abdeckeinrichtung. Hierdurch können besonders bevorzugt in der Abdeckung bereits befindliche Durchbrechungen, Öffnungen oder Löcher freigelegt werden. Dies ist einer definierten Abgabe und Zerstäubung der Formulierung zuträglich.

Die Formulierung kann vorschlagsgemäß besonders wirkungsvoll insbesondere aus einer entsprechenden Aufnahme des Trägers ausgegeben, dispergiert und/oder ausgetragen werden.

Zum Austrag der Formulierung kann wahlweise der Luftstrom zum Bewegen des Trägers oder ein sonstiger Luftstrom eingesetzt werden.

Bei der vorliegenden Erfindung sind die Begriffe "Luft" und "Luftstrom" vorzugsweise in einem weiteren Sinne auch dahingehend zu verstehen, dass auch ein sonstiges Gas bzw. eine Strömung eines sonstigen Gases umfasst ist. Nachfolgend wird jedoch immer der Begriff "Luft" verwendet, da üblicherweise Luft als Gas zum Antrieb bzw. zur Bewegung des Trägers und/oder als Fördermedium zur Förderung der Formulierung nach dem Lösen von oder aus dem Träger bzw. zum Dispergieren der Formulierung eingesetzt wird. Vorzugsweise wird der Luftstrom beim Inhalieren bzw. durch ein Einatmen erzeugt. Es handelt sich also insbesondere um einen passiven Inhalator.

Besonders bevorzugt ist der Inhalator zur Ausgabe bzw. Verabreichung oder Inhalation lediglich einer Einzeldosis ausgebildet. Besonders bevorzugt handelt es sich also um ein Einweggerät. Jedoch kann der Inhalator alternativ auch zur mehrfachen Verwendung und/oder zur getrennten Ausgabe mehrerer Dosen der Formulierung ausgebildet sein. Beispielsweise sind dann mehrere Aufnahmen vorgesehen, die unabhängig voneinander öffenbar sind.

Die vorgenannten Aspekte und die sich aus der weiteren Beschreibung und den Ansprüchen ergebenden Aspekte können auch unabhängig voneinander, aber auch in beliebiger Kombination realisiert werden.

Einzelne Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt eines vorschlagsgemäßen Inhalators gemäß einer ersten Ausführungsform;
- Fig. 2: einen schematischen horizontalen Schnitt des Inhalators gemäß Fig. 1 entlang Linie II-II;
- Fig. 3: einen schematischen Schnitt eines vorschlagsgemäßen Inhalators gemäß einer zweiten Ausführungsform;
- Fig. 4: einen schematischen Schnitt eines vorschlagsgemäßen Inhalators gemäß einer dritten Ausführungsform mit verschlossener Abdeckung einer Aufnahme eines Trägers; und
- Fig. 5: einen schematischen Schnitt des Inhalators gemäß Fig. 4 mit geöffneter Abdeckung.

In den Figuren werden für gleiche oder ähnliche Teile die gleichen Bezugszeichen verwendet, auch wenn eine wiederholte Beschreibung weggelassen ist. Insbesondere ergeben sich dann auch die gleichen oder entsprechende Vorteile und Eigenschaften. Die einzelnen Figuren sind aus Darstellungs- oder Vereinfachungsgründen nicht maßstabsgerecht und auf wesentliche für die Erfindung relevante Komponenten reduziert.

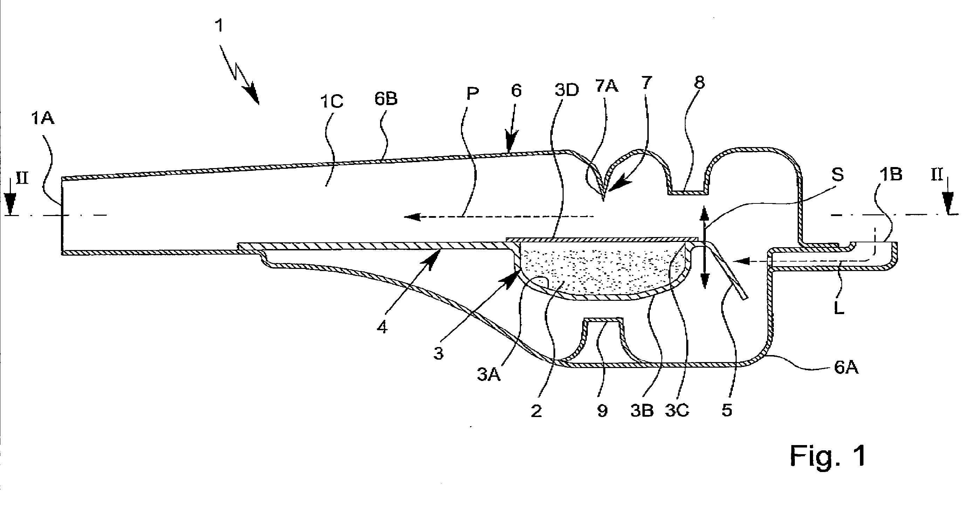

Fig. 1 zeigt schematisch den Aufbau eines vorschlagsgemäßen Inhalators 1 gemäß einer ersten Ausführungsform. Der Inhalator 1 ist vorzugsweise tragbar ausgebildet und/oder arbeitet insbesondere nur mechanisch.

Der Inhalator 1 dient der Ausgabe bzw. Inhalation einer vorzugsweise pulverförmigen Formulierung 2 im eingangs genannten Sinne. Die Formulierung 2 ist vorzugsweise vordosiert in einer einzelnen Dosis oder mehrere Dosen.

Beim Darstellungsbeispiel ist die Formulierung 2 vorzugsweise vordosiert, also außerhalb des Inhalators 1 bereits in einer einzelnen Dosis oder in mehrere Dosen vordosiert. Grundsätzlich ist es jedoch auch möglich, die Formulierung 2 erst unmittelbar vor jeder Inhalation und/oder im Inhalator 1 zu dosieren.

Ein Träger 3 trägt die Formulierung 2 bzw. stellt diese bereit. Insbesondere weist der Träger 3 mindestens eine Vertiefung bzw. Aufnahme 3A o. dgl. zum Aufnehmen insbesondere einer Dosis der Formulierung 2 auf. Jedoch sind hier auch andere konstruktive Lösungen möglich. Beispielsweise kann die Formulierung 2 auf der Oberfläche oder in einem Bereich der Oberfläche oder auf der gesamten Oberfläche des Trägers 3 angeordnet oder verteilt sein.

Der Träger 3 ist insbesondere flexibel, flach, bandförmig, streifenförmig, blisterartig und/oder folienartig ausgebildet. Er ist aus einem geeigneten Material, insbesondere aus Kunststoff, Metallfolie, einem Verbundmaterial o. dgl., hergestellt oder aufgebaut.

Der Träger 3 kann nur eine Aufnahme 3A oder mehrere Aufnahmen 3A aufweisen. Jede Aufnahme 3A kann bedarfsweise von einer optionalen Abdeckung 3D, wie in Figur 1 beispielhaft angedeutet, abgedeckt sein. Zum Austrag der Formulierung 2 ist die Abdeckung 3D dann vorzugsweise öffenbar, abziehbar oder dergleichen. Beispielsweise kann die Abdeckung 3D aufgeschnitten, aufgerissen, perforiert oder in sonstiger Weise zumindest teilweise entfernt und/oder geöffnet werden, um einen Austrag der Formulierung 2 zu ermöglichen. Besonders bevorzugt erfolgt das Öffnen durch eine zugeordnete Öffnungseinrichtung 7, ggfs. auch erst bei Bewegung des Trägers 3. Auf die Öffnungseinrichtung wird später noch eingegangen.

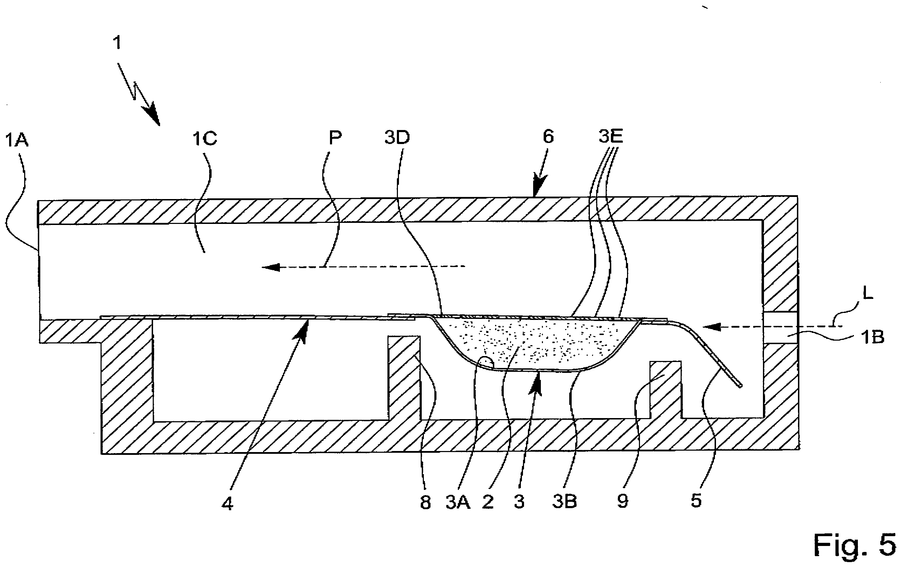

Die Abdeckung 3D und/oder eine oder mehrere verhältnismäßig kleine Austrittsöffnungen, Durchbrechungen oder Löcher 3E (siehe Fig. 5) oder dergleichen führt bzw. führen besonders bevorzugt dazu, dass die Formulierung 2 verhältnismäßig langsam - insbesondere mit einer gewünschten Rate - abgegeben und ausgetragen wird.

Der Träger 3 ist, wie in Fig. 1 schematisch angedeutet, vorzugsweise blisterartig ausgebildet. Insbesondere weist der Träger 3 ein Trägerelement bzw. Bodenelement 3B auf, das insbesondere eine Vertiefung aufweist bzw. tiefgezogen ist, um die Aufnahme 3A bzw. einen Aufnahmeraum für die Formulierung 2 zu bilden. Der Aufnahmeraum bzw. die Aufnahme 3A ist vorzugsweise von der Abdeckung 3D abgedeckt.

Insbesondere ist das Trägerelement bzw. Bodenelement 3B durch eine Folie oder ein Folienmaterial oder Verbundmaterial gebildet.

Die Abdeckung 3D ist vorzugsweise auf das Trägerelement bzw. Bodenelement 3B aufgesiegelt oder auflaminiert.

Besonders bevorzugt ist die Abdeckung 3D durch eine dünne Folie, beispielsweise aus Kunststoff, Aluminium, einem Verbundmaterial oder dgl. gebildet.

Besonders bevorzugt bildet der Träger 3 bzw. dessen Trägerelement/Bodenelement 3B und Abdeckung 3D einen Blister oder eine Blisterverpackung.

Der Inhalator 1 ist beim Darstellungsbeispiel vorzugsweise als Einweginhalator und/oder zur Ausgabe bzw. Verabreichung nur einer Dosis ausgebildet. Jedoch kann es sich grundsätzlich auch um einen Mehrweginhalator handeln, der vorzugsweise zur unabhängigen Ausgabe von mehreren Dosen der Formulierung oder verschiedener Formulierungen ausgebildet ist.

Bei einer Ausbildung als Mehrweginhalator kann der Träger 3 mehrere unabhängige voneinander öffenbare Aufnahmen 3A aufweisen und/oder fingerartige Vorsprünge mit jeweils einer Aufnahme 3A aufweisen. Die verschiedenen Aufnahmen 3A bzw. Fingerbereiche können insbesondere in einem Endbereich miteinander bzw. derart verbunden sein, dass die Fingerbereiche bzw. Aufnahmen 3A unabhängig voneinander in Schwingung versetzbar sind. Der Träger 3 ist dann beispielsweise quer zur Strömungsrichtung und/oder Schwingungsrichtung bewegbar oder durch den Inhalator 1 ziehbar, insbesondere um von einer Aufnahme 3A zur nächsten Aufnahme 3A zu wechseln bzw. um den jeweils nächsten Fingerbereich in eine Position zu bringen, um dass vorschlagsgemäße Schwingen und Ausbringen der jeweiligen Dosis aus der jeweiligen Aufnahme 3A zu ermöglichen.Der Inhalator 1 weist vorzugsweise mindestens einen Träger 3 auf oder ist zur Aufnahme mindestens eines Trägers 3 ausgebildet.

Wie bereits erwähnt, kann der Träger 3 auch mehrere Aufnahmen 3A für mehrere Dosen, ggf. auch für Dosen verschiedener Formulierungen 2, aufweisen. So ist es beispielsweise möglich, zwei unterschiedliche Formulierungen 2, die in unterschiedlichen Aufnahmen 3A bereitgehalten werden, gemeinsam über den Luftstrom L auszutragen und dabei ggf. zu mischen und/oder zumindest gleichzeitig oder ggf. auch zeitlich nacheinander einem Patienten beim Inhalieren zuzuführen.

Beim Darstellungsbeispiel ist der Träger 3 vorzugsweise fest im Inhalator 1 gehalten oder angeordnet und/oder nicht wechselbar. Der Inhalator 1 weist daher beim Darstellungsbeispiel vorzugsweise einen Träger 3 auf.

Der Träger 3 ist vorzugsweise über einen Federabschnitt 4 beweglich gehalten. Insbesondere ist der Träger 3 zumindest im Wesentlichen quer oder senkrecht zu seiner Flächenerstreckung beweglich vom Federabschnitt 4 gehalten. Besonders bevorzugt ist der Federabschnitt 4 zur Schwingung des Trägers 3 federelastisch verformbar. Alternativ oder zusätzlich kann auch der Träger 3 federelastisch verformbar ausgebildet sein.

Der Träger 3 bzw. dessen Trägerelement oder Bodenelement 3B ist vorzugsweise zumindest im Wesentlichen flach ausgebildet oder durch eine Hauptflächenerstreckung (bei der Darstellung gemäß Fig. 1 horizontal und senkrecht zur Zeichenebene bzw. durch eine Ebene parallel zur Schnittebene II-II von Fig. 1) charakterisiert.

Der Federabschnitt 4 erstreckt sich vorzugsweise zumindest im Wesentlichen in der Haupterstreckungsebene des Trägers 3 oder in einer dazu parallelen Ebene.

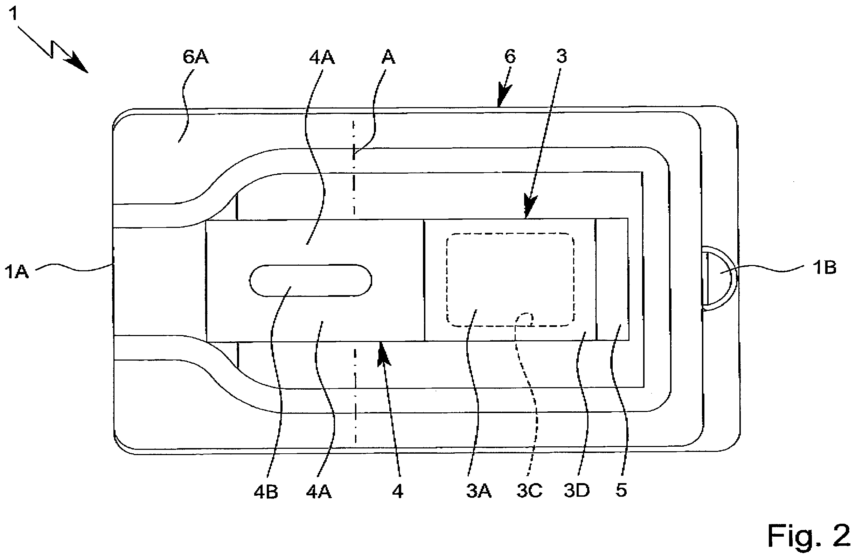

Besonders bevorzugt bildet der Federabschnitt 4 eine (virtuelle) Achse A (vgl. Fig. 2), um die sich der Träger 3 insbesondere rotatorisch bewegen kann. Hierbei wir der Träger 3 von dem Federabschnitt 4 geführt bzw. gehalten. Die Achse A liegt dabei nicht fest, sondern kann insbesondere in der Ebene des Federabschnitts 4 (insbesondere je nach Material und Form des Federabschnitts 4) variieren oder wandern und/oder sich auch in anderen Ebenen translatorisch bewegen. Der Federabschnitt 4 ist insbesondere aufgrund des bevorzugten Aufbaus insbesondere derart ausgebildet, dass die Achse A nicht verkippt, sondern sich nur parallel verschiebt.

Der Federabschnitt 4 ist vorzugsweise zumindest im Wesentlichen stegartig oder blattartig ausgebildet. Der Federabschnitt 4 ist vorzugsweise zumindest im Wesentlichen bandförmig und/oder folienartig ausgebildet.

Dem Horizontalschnitt gemäß Fig. 2 ist zu entnehmen, dass der Federabschnitt 4 zwei Federbereiche oder -stege 4A und mindestens eine Durchbrechung, vorzugsweise einen insbesondere dazwischen angeordneten Schlitz bzw. Längsschlitz 4B aufweist. Versuche haben gezeigt, dass sich so ein sehr definiertes Feder- und Biegeverhalten erreichen lässt. Dies ist einer definierten Ausgabe der Formulierung 2 und Bewegung, Amplitude und/oder Frequenz des Trägers 3 sehr zuträglich.

Weiter verhindert die Durchbrechung, dass der Federabschnitt 4 und Träger 3 bei Erreichen einer Endlage den Strömungsweg ventilartig verschließen können. Vielmehr kann durch die Durchbrechung dann weiter Luft strömen.

Vorzugsweise beträgt die Größe der Durchbrechung mindestens 10%, insbesondere etwa 20% oder mehr der Gesamtfläche des Federabschnitts 4 und/oder weniger als 70%, besonders bevorzugt weniger als 50% der Gesamtfläche des Federabschnitts 4.

Beim Darstellungsbeispiel ist die Durchbrechung vorzugsweise schlitzartig ausgebildet. Jedoch kann die Durchbrechung auch jede sonstige Form aufweisen.

Weiter können auch mehrere Durchbrechungen vorgesehen sein.

Der Federabschnitt 4 kann von einem separaten Materialstück oder, wie bei der ersten Ausführungsform angedeutet, von dem Trägerelement bzw. Bodenelement 3B des Trägers 3 gebildet sein.

Der Federabschnitt 4 kann einstückig mit dem Träger 3 ausgebildet und/oder aus dem gleichen Material hergestellt sein. Das Material im Bereich des Federabschnitts 4 kann zur besserem Elastizität beispielsweise auch dünner ausgebildet und/oder gezogen sein. Alternativ oder zusätzlich kann die Elastizität des Federabschnitts 4 auch durch entsprechende Anordnung und/oder Größe der Durchbrechung 3 beeinflusst oder festgelegt werden.

Alternativ oder zusätzlich kann der vorzugsweise blattförmige Federabschnitt 4 auch aus einem anderen Material, beispielsweise einem elastischen Kunststoff, insbesondere Polycarbonat, oder aus metallischem Federblech hergestellt sein.

Der Träger 3 ist mit dem Federabschnitt 4 vorzugsweise durch Kleben oder Schweißen verbunden, jedoch ist auch ein Anspritzen oder Überspritzen zum Verbinden möglich. Generell kann die Verbindung auf jede geeignete Art und Weise erfolgen.

Der Federabschnitt 4 ist beim Darstellungsbeispiel an einem Ende vorzugsweise fest am Inhalator 1 oder einem Gehäuse 6 des Inhalators 1 angebracht oder gehalten, vorzugsweise durch Kleben, Schweißen Einklemmen oder dergleichen. Am anderen bzw. freien Ende des Federabschnitts 4 schließt sich vorzugsweise der Träger 3 - insbesondere in Verlängerung - an.

Besonders bevorzugt ist dem Träger 3 ein Flügel 5 zugeordnet, wie in Fig. 1 und 2 angedeutet. Der Flügel 5 ist vorzugsweise an dem Federabschnitt 4 abgewandten Ende bzw. freien Ende des Trägers 3 angeordnet.

Der Flügel 5 ist vorzugsweise zu der Flächenerstreckung des Trägers 3 und/oder Richtung des anströmenden Luftstroms L geneigt. Die Neigung des Flügels 5 zu der Flächenerstreckung des Trägers 3 bzw. zur Luftströmung beträgt vorzugsweise zumindest im wesentlichen 30° bis 70° insbesondere im wesentlichen 40° bis 50°, ganz besonders bevorzugt im wesentlichen 45°.

Der Flügel 5 ist vorzugsweise einstückig mit dem Träger 3 ausgebildet oder von dem (gleichen) Trägermaterial gebildet, insbesondere durch entsprechende Umformung. Alternativ kann der Flügel 5 auch aus einem sonstigen Material oder Materialstück, beispielsweise einem Federblech, hergestellt sein, das am Träger 3 befestigt ist.

Vorzugsweise ist ein weicher oder gerundeter Übergang, insbesondere mit einem Radius von mindestens 1 bis 2 mm, zwischen dem Träger 3 und dem Flügel 5 vorgesehen.

Der Flügel 5 ist vorzugsweise möglichst nahe an der Aufnahme 3A, insbesondere also unmittelbar benachbart zu dieser, angeordnet.

Der Träger 3 ist mittels des Luftstroms L zum Austrag der Formulierung 2 - also zum Lösen der Formulierung 2 vom Träger 3 bzw. aus der Aufnahme 3A - bewegbar, insbesondere in Schwingung versetzbar. Der Luftstrom L kann unmittelbar und/oder mittelbar auf den Träger 3 einwirken bzw. diesen bewegen.

Zur Schwingung des Trägers 3 wird der Federabschnitt 4 vorzugsweise federelastisch verformt. Insbesondere schwingt auch der Federabschnitt 4. Vorzugsweise bildet die Luftströmung L auch ein Fördermedium, um die vom Träger 3 insbesondere durch dessen Bewegung gelöste Formulierung 2 zu dispergieren und/oder zu fördern und vorzugsweise über ein Mundstück bzw. einen Auslass 1A des Inhalators 1 auszutragen und an einen nicht dargestellten Benutzer auszugeben. Jedoch kann als Fördermedium für die Formulierung 2 auch ein anderer bzw. getrennter Luftstrom bzw. sonstige Luft im eingangs genannten Sinne - also auch sonstiges Gas - eingesetzt werden.

Beim Darstellungsbeispiel wird die Luftströmung L vorzugsweise durch das Inhalieren bzw. Einatmen des nicht dargestellten Benutzers erzeugt, und zwar insbesondere durch Ansaugen von Umgebungsluft.

Der angesaugte bzw. in den Inhalator 1 bzw. dessen Gehäuse 6 durch den Einlass 1B einströmende Luftstrom L trifft vorzugsweise zuerst auf den schrägstehenden Flügel 5. Der optionale Flügel 5 kann die bevorzugte Querbewegung bzw. Schwingung S des Trägers 3 verursacht durch den Luftstrom L unterstützen oder bewirken.

Der Träger 3 wird durch den Luftstrom L ausgelenkt und in Schwingung S versetzt. Die Bewegungsebene läuft bei der Darstellung gemäß Fig. 1 vorzugsweise in der Zeichenebene. Insbesondere führt der Träger 3 dabei eine rotatorische- oder Schwenkbewegung, geführt von dem Federabschnitt 4 bzw. um die Achse A (Fig. 2) aus,

Zum Öffnen der Aufnahme 3A bzw. Abdeckung 3D weist der Inhalator 1 vorzugsweise die Öffnungseinrichtung 7 auf. Die Öffnungseinrichtung 7 ist insbesondere zum Anstechen der Abdeckung 3D ausgebildet. Hierzu weist die Öffnungseinrichtung 7 vorzugsweise mindestens ein Anstechelement 7A auf.

Bei der ersten Ausführungsform ist die Öffnungseinrichtung 7 bzw. das Anstechelement 7A vorzugsweise durch einen Wandungsbereich des Inhalators 1 bzw. des Gehäuses 6 des Inhalators 1, insbesondere eines Oberteils 6B des Gehäuses 6, gebildet oder daran angeordnet.

Vorzugsweise ist die Öffnungseinrichtung 7 bzw. das Anstechelement 7A bzw. der Wandungsbereich manuell eindrückbar, so dass das Abdeckelement 7A die Abdeckung 3D anstechen kann, wobei dieses Anstechen beispielsweise in der in Fig. 1 gezeigten Ausgangs- oder Mittenlage des Trägers 3 und/oder bei Auslenkung des Trägers 3, beispielsweise in einer Endlage, in der der Träger 3 bzw. dessen Bodenelement 3B beispielsweise an einem gegenüberliegenden Anschlag 9 anschlägt, erfolgen kann.

Es ist alternativ jedoch auch möglich, dass die Aufnahme 3A bzw. Abdeckung 3D alleine durch das Bewegen bzw. Schwingen des Trägers 3 geöffnet wird. In diesem Fall muss der Wandungsbereich nicht flexibel ausgebildet sein. Stattdessen ist die Öffnungseinrichtung 7 bzw. deren Anstechelement 7A dann vorzugsweise so angeordnet, dass bei entsprechendem Schwingen des Trägers 3 automatisch ein Anstechen erfolgt.

Nach dem Öffnen der Aufnahme 3A bzw. der Abdeckung 3D wird beim weiteren Schwingen des Trägers 3 die Formulierung 2 durch die Bewegung und/oder den Luftstrom L ausgetragen. Je nach Öffnen der Abdeckung 3D kann der Luftstrom L auch durch die Aufnahme 3A strömen, um die Formulierung 2 auszutragen,

Der Luftstrom L nimmt das Pulver mit, so dass ein pulverbeladener Luftstrom P entsteht, wie schematisch in Fig. 1 angedeutet.

Der Luftstrom L bzw. pulverbeladene Luftstrom P strömt vorzugsweise zumindest im wesentlichen ausschließlich auf einer Flachseite, hier der oberen Flachseite bzw. abdeckseitigen Flachseite des Trägers 3 bzw. der die Formulierung 2 abgebenden Flachseite de Trägers 3 vorbei oder entlang.

Der pulverbeladene Luftstrom P kann vorzugsweise zumindest im wesentlichen geradlinig zu dem Auslass 1A des Inhalators 1 strömen und darüber an einen nicht dargestellten Benutzer ausgegeben werden.

Das Gehäuse 6 des Inhalators 1 ist beim Darstellungsbeispiel vorzugsweise aus dem Unterteil 6A und dem Oberteil 6B bzw. zwei Hälften oder Teilen aufgebaut. Besonders bevorzugt ist das Gehäuse 6 zweiteilig bzw. zweischalig oder aus zwei Halbschalen aufgebaut. Die beiden Teile bzw. das Unterteil 6A und das Oberteil 6B werden vorzugsweise miteinander verschnappt, verklebt, verschweißt und/oder auf sonstige geeignete Weise miteinander verbunden. Alternativ kann das Gehäuse 6 bzw. können diese Teile 6A, 6B auch einteilig ausgebildet oder durch ein zusammenklappbares Teil, dass besonders bevorzugt in einem Kunststoff-Spritzgussvorgang hergestellt ist, gebildet sein. Jedoch sind auch andere konstruktive Lösungen möglich.

Der Inhalator 1, das Gehäuse 6 und/oder der Träger 3 bzw. die Aufnahme 3A ist bzw. sind vorzugsweise - zumindest partiell - transparent ausgebildet. So kann ein Nutzer sehr leicht erkennen, ob der Inhalator 1 bereits benutzt bzw. entleert wurde oder noch verwendet werden kann.

Besonders bevorzugt weist der Inhalator 1 ein Mundstück auf, das beim Darstellungsbeispiel vorzugsweise durch das Gehäuse 6 und/oder im Bereich des Auslasses 1A gebildet ist. Dementsprechend kann der nicht dargestellte Benutzer den Inhalator 1 vorzugsweise direkt in den Mund nehmen und einatmen, um Umgebungsluft anzusaugen und den Luftstrom L zu erzeugen.

Der Inhalator 1 ist vorzugsweise derart ausgebildet, dass bei einem Luftstrom L von 70 l/min. ein Druckabfall von etwa 3 bis 5 kPa, besonders bevorzugt von im wesentlichen 4 kPa, auftritt.

Der Inhalator 1 ist vorzugsweise für einen Volumenstrom von etwa 20 bis 100 l/min. ausgebildet.

Der Inhalator 1 ist vorzugsweise derart ausgebildet, dass die Frequenz der Schwingung zumindest im wesentlichen 50 bis 100 Hz, besonders bevorzugt 60 bis 90 Hz und ganz besonders bevorzugt im wesentlichen 70 bis 80 Hz, beträgt, insbesondere bei einem Volumenstrom von 20 bis 100 l/min., besonders bevorzugt bei einem Volumenstrom von im wesentlichen 70 l/min.

Die Frequenz der Schwingung hängt insbesondere von der Füllmenge bzw. dem Füllgewicht ab. Insbesondere wird die Füllmenge der Aufnahme 3A so gewählt, dass eine gewünschte Frequenz erreicht wird. Ausgehend von einer bestimmten Dosis oder Wirkstoffmenge kann der Formulierung beispielsweise zur Erhöhung des Gewichts ein zusätzlicher Stoff oder ein größerer Anteil eines bestimmten Stoffs zugesetzt werden, um die Frequenz zu beeinflussen.

Der Inhalator 1 ist vorzugsweise derart ausgebildet, dass die Amplitude der Schwingung mindestens 1 mm, insbesondere im wesentlichen 2 mm beträgt und/oder weniger als 10 mm, vorzugsweise weniger als 6 mm, insbesondere weniger als 4 mm, beträgt.

Der Einlass 1B für die Luft bzw. den Luftstrom L ist vorzugsweise auf der dem Auslass 1A gegenüberliegenden Seite und/oder benachbart zu dem freien Ende des Trägers 3 bzw. zu dem Flügel 5 angeordnet.

Insbesondere ist die Richtung des durch den Einlass 1B einströmenden Luftstroms L beim Auftreffen auf den Träger 3 bzw. Flügel 5 zumindest im wesentlichen parallel zu der Hauptströmungsrichtung des pulverbeladenen Luftstroms P bzw. zu der Hauptaustrittsrichtung oder Hauptausgaberichtung des Inhalators 1, also bei der Darstellung gemäß Fig. 1 zumindest im wesentlichen horizontal.

Der Lufteinlass 1B ist vorzugsweise zwischen bzw. von den beiden Gehäuseteilen 6A und 6B gebildet.

Der Inhalator 1 bzw. dessen Gehäuse 6 bildet vorzugsweise einen Strömungsraum 1C, der zumindest im wesentlichen absatzfrei und/oder geradlinig von dem Träger 3 bzw. dessen Aufnahme 3A zu dem Auslass 1A verläuft, wobei sich der Querschnitt des Strömungsraums 1C, insbesondere zum Auslass hin 1A, verringern kann, beim Darstellungsbeispiel parallel zur Haupterstreckungsrichtung bzw. Flächenerstreckung des Trägers 3, wie in Fig. 2 angedeutet, um auslassseitig ein Mundstück bzw. einen Mundstückbereich zu bilden. Jedoch sind auch andere konstruktive Lösungen möglich.

Besonders bevorzugt wird der Träger 3 zumindest im wesentlichen nur im Bereich seines freien Endes und/oder nur auf einer Flachseite angeströmt. Zur Begrenzung der Schwingung S des Trägers 3 weist der Inhalator 1 vorzugsweise mindestens einen, beim Darstellungsbeispiel zwei oder mehr Anschläge 8 und 9 auf, wie in Fig. 1 dargestellt.

Die Anschläge 8 und 9 sind beim Darstellungsbeispiel vorzugsweise von dem Gehäuse 6 bzw. deren Wandung gebildet. Jedoch sind auch andere konstruktive Lösungen möglich.

Die Anschläge 8 und 9 begrenzen die Amplitude bzw. Auslenkung des Trägers 3 beim Schwingen. Dies ist einem definierten Schwingen und damit einer definierten Austragung der Formulierung 2 zuträglich.

Der Träger 3 bzw. dessen Trägerelement oder Bodenelement 3B ist vorzugsweise aus einem Verbundmaterial von Aluminium, Polyamid, PVC und Siegellack oder dergleichen gebildet oder aufgebaut.

Der Federabschnitt 4 kann aus dem gleichen Material oder einem anderen Material hergestellt sein. Besonders bevorzugt ist der Federabschnitt 4 aus einem elastischen Kunststoff, insbesondere Polycarbonat, hergestellt.

Gemäß einem besonders bevorzugten, auch unabhängig realisierbaren Aspekt weist der Träger 3 bzw. die Aufnahme 3A einen besonders bzw, zumindest im wesentlichen eckigen Übergang der Kante 3C des Trägerelements bzw. Bodenelements 3B zu der Abdeckung 3D auf, wie in Fig. 1 schematisch angedeutet. Hierdurch wird ein spitzer Zwickel in diesem Bereich vermieden da sich ansonsten dort die Formulierung 2 in unerwünschter Weise absetzen oder festsetzen kann. Der bevorzugte zumindest im wesentlichen rechteckige Übergang von der Seitenwandung bzw. Kante 3C zu der Abdeckung 3D kann einem solchen Festsetzen nämlich entgegenwirken und/oder ist einem zumindest im wesentlichen vollständigen Austragen der Formulierung 2 zuträglich.

Normalerweise wird der Träger 3 bzw. dessen Trägerelement oder Bodenelement 3B aus einer dickeren Folie, aus Verbundmaterial oder dergleichen durch Heißprägen, Blasformen oder Tiefziehen hergestellt. Um die vorzugsweise eckige oder rechtwinklige Kante 3C beim Übergang zur Abdeckung 3D realisieren zu können, ist eine Herstellung durch Kunststoff-Spritzguss bevorzugt.

Es ist grundsätzlich möglich, gleichzeitig mehrere Dosen und/oder unterschiedliche Formulierungen 2 - bedarfsweise auch aus unterschiedlichen Aufnahmen 3A - auszutragen. Der Träger 3 kann hierzu beispielsweise mehrere Aufnahmen 3A mit ggf. unterschiedlichen Formulierungen 2 aufweisen. Hier ist dann eine vorzugsweise parallele Anordnung der Aufnahmen 3A bevorzugt, so dass zumindest im wesentlichen gleiche Schwingungen oder Schwingungseigenschaften erreicht werden. Jedoch sind auch andere Konfigurationen möglich.

Das vorzugsweise vorgesehene Dispergierverfahren (Bewegung des Trägers 3 bzw. der geöffneten oder sich öffnenden Aufnahme 3A im Luftstrom L zur Ausgabe und Dispersion der Formulierung 2 im Luftstrom L) hat verschiedene Vorteile, Es wird eine sehr gleichmäßige Dispersion mit guter Deagglomaration der Partikel der vorzugsweise pulverförmigen Formulierung 2 ermöglicht. Es wird eine relative Volumenstromunabhängigkeit ermöglicht. Insbesondere beginnt die Ausgabebewegung bzw. -vibration oder -Schwingung und damit die Ausgabe der Formulierung 2 vorzugsweise erst ab etwa einer Flussrate bzw. einem Volumenstrom von 10 oder 20 l/min. Weiter kann ein für den Benutzer wahrnehmbares akustisches Signal durch das Schwingen - also beim Inhalieren - erzeugt werden.

Nachfolgend werden weitere Ausführungsformen anhand der weiteren Figuren näher erläutert. Hierbei werden insbesondere nur wesentliche Unterschiede oder neue Aspekte näher erläutert. Die bisherigen Ausführungen und Erläuterungen gelten daher insbesondere ergänzend oder entsprechend.

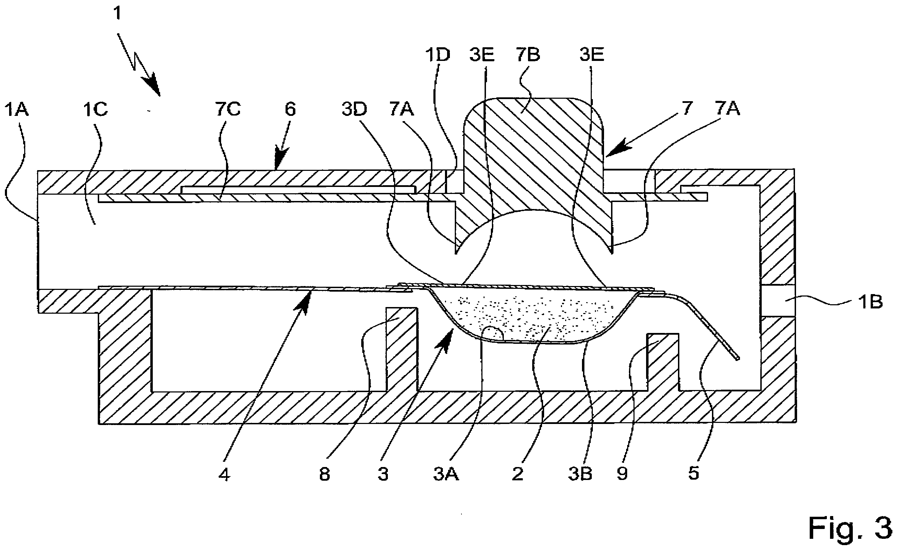

Fig. 3 zeigt eine zweite Ausführungsform des vorschlagsgemäßen Inhalators1 in einem schematischen Ausschnitt ähnlich wie Fig. 1.

Bei der zweiten Ausführungsform sind die Anschläge 8 und 9 vorzugsweise nur auf einer Seite angeordnet.

Bei der zweiten Ausführungsform können vorzugsweise in Strömungsrichtung hintereinander liegende Durchbrechungen, Öffnungen oder Löcher 3E in der Abdeckung 3D erzeugt werden. Bei der zweiten Ausführungsform weist die Öffnungseinrichtung 2 nämlich in Strömungsrichtung hintereinander liegende bzw. mehrere Anstechelemente 7A auf. Die Öffnungseinrichtung 7 ist also vorzugsweise zur Zeugung mehrerer Öffnungen bzw. Löcher 3E ausgebildet. Dies ist einer besonders guten Entleerung des Trägers 3 bzw. der Aufnahme 3A bzw. einem guten oder vollständigen Austrag der Formulierung 2 zuträglich.

Die Öffnungseinrichtung 7 weist vorzugsweise ein Betätigungselement 7B zur manuellen Betätigung der Öffnungseinrichtung 7 auf. Beim Darstellungsbeispiel weist der Inhalator 1 bzw. das Gehäuse 6 vorzugsweise eine Durchbrechung 1D auf, durch die sich das Betätigungselement 7B nach außen erstreckt. Durch Eindrücken des Betätigungselements 7B ist die Öffnungseinrichtung 7 betätigbar und der Träger 3 bzw. die Abdeckung 3D mittels der Anstechelemente 7A öffenbar bzw. anstechbar.

Vorzugsweise weist die Öffnungseinrichtung 7 ein Federelement 7C auf, dass das mindestens eine Anstechelement 7A und das Betätigungselement 7B federnd hält, insbesondere in der in Fig. 3 gezeigten Ausgangslage, aus der die Anstechelemente 7A durch Drücken auf das Betätigungselement 7B von außen in eine zum Träger 3 hin vorgerückte Lage (in Fig. 3 nach unten) bewegbar ist. Nach dem Loslassen des Betätigungselements 7B sorgt eine vorzugsweise elastische Rückstellung durch das Federelement 7C dafür, dass die Ausgangslage wieder eigenommen und insbesondere die Durchbrechung 1D wieder verschlossen wird.

Jedoch sind auch andere konstruktive Lösungen möglich.

Bei der zweiten Ausführungsform ist schematisch angedeutet, dass der Federabschnitt 4 aus einem anderen Material oder Materialstück als der Träger 3 bzw. dessen Trägerelement 3A oder Bodenelements 3B hergestellt ist.

Der Flügel 5 kann ebenfalls aus einem anderen Material oder Materialstück hergestellt sein. Der Flügel 5 ist dann vorzugsweise noch in geeigneter Weise mit dem Träger bzw. dessen Trägerelement oder Bodenelements 3B verbunden.

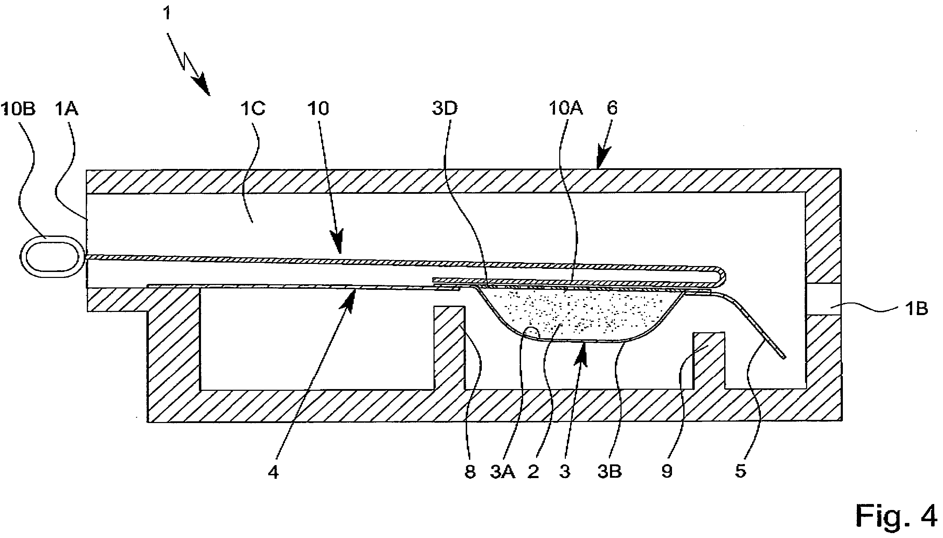

Nachfolgend wird eine dritte Ausführungsform anhand der Fig. 4 und 5 erläutert. Fig. 4 zeigt den vorschlagsgemäßen Inhalator 1 in einem zu Fig. 1 korrespondierenden schematischen Schnitt mit einem durch eine Abdeckeinrichtung 10 verschlossenen oder abgedeckten Träger 3. Fig. 5 zeigt den Inhalator 1 in einem korrespondierenden Schnitt, jedoch bei entfernter Abdeckeinrichtung 10.

Der Inhalator 1 gemäß der dritten Ausführungsform weist vorzugsweise die Abdeckeinrichtung 10 auf, um die Aufnahme 3A bzw. deren Abdeckung 3D abzudecken. Die Abdeckeinrichtung 10 bzw. ein Abdeckelement 10A der Abdeckeinrichtung 10 ist zum Öffnen der Aufnahme 3A bzw. von einer oder mehreren Durchbrechungen oder Löchern 3E der Abdeckung 3D abziehbar oder entfernbar.

Besonders bevorzugt weist die Abdeckeinrichtung 10 ein insbesondere folienartiges Abdeckelement 10A auf, dass den Träger 3 bzw. die Aufnahme 3A bzw. die Abdeckung 3D bzw. in der Abdeckung 3D vorgesehene Durchbrechungen oder Löcher 3E abdeckt, wie in Fig. 4 schematisch angedeutet. Fig. 4 zeigt also den verschlossenen Zustand.

Das Abdeckelement 10A ist vorzugsweise umgelegt, umgeklappt bzw, umgefaltet.

Das Abdeckelement 10A und/oder ein optional daran angeordnetes Griffelement 10B der Abdeckeinrichtung 10 ragt vorzugsweise aus dem Inhalator 1 bzw. dessen Gehäuse 6, insbesondere aus dem Auslass 1A heraus.

Durch Ziehen an dem Abdeckelement 10A oder Griffelement 10B kann das Abdeckelement 10A vorzugsweise abgezogen bzw. entfernt werden. Dies geht aufgrund des Umklappens besonders leicht, da sich das Abdeckelement 10A im Bereich der Umbiegung oder Umfaltung beim Ziehen quasi abrollt und so relativ leicht von dem Träger 3 bzw. der Abdeckung 3D löst bzw. abzieht.

Das Griffelement 10B bzw. das Abdeckelement 10A weist vorzugsweise eine für den Benutzer leicht erkennbare Farbe oder Signalfarbe, insbesondere rot, auf. Dementsprechend ist es für den Benutzer intuitiv klar, dass er zunächst daran ziehen muss, also zunächst das Abdeckelement 10A entfernen muss, bevor er den Inhalator 1 benutzen kann.

Nach dem Lösen des Abdeckelements 10A bzw. Entfernen des Abdeckelements 10A bzw. der Abdeckeinrichtung 10 ist die Aufnahme 3A bzw. sind die Durchbrechungen bzw. Löcher 3E geöffnet, wie in Fig. 5 dargestellt, so dass der Inhalator 1 direkt benutzt werden kann.

Wenn die Abdeckung 3D nur ein Loch 3E aufweist, ist dieses vorzugsweise in der Nähe des Flügels 5 angeordnet. Bei zwei oder mehr Löchern 3 sind diese vorzugsweise in gegenüberliegenden Endbereichen in Längsrichtung bzw, Luftströmungsrichtung gesehen angeordnet.

Mehrere Löcher 3E haben den Vorteil, dass sich die Verstopfung eines Lochs nicht sehr stark bzw. geringer auf die Zerstäubung bzw. Austragung der Formulierung 2 auswirkt.

Der Inhalator 1 bzw. Träger 3 bzw. die Abdeckung 3D weist vorzugsweise mehrere Durchbrechungen oder Löcher 3E, besonders bevorzugt 3 bis 5 Durchbrechungen oder Löcher 3E, auf.

Die Durchbrechungen bzw. Löcher 3E sind vorzugsweise rund ausgebildet und/oder weisen vorzugsweise einen Durchmesser von 0,5 bis 3 mm, insbesondere im wesentlichen 1,0 bis 2,7 mm, ganz besonders bevorzugt von etwa 1,5 mm auf.

Die Löcher 3E müssen nicht rund sein, sondern können jede sonstige Form aufweisen, beispielsweise auch dreieckig ausgebildet sein, insbesondere passend zu dem jeweiligen Öffnungsmechanismus bzw. angepasst an die Form der Aufnahme 3A.

Die Ausbildung von vorbestimmten bzw. definierten, insbesondere vorschlagsgemäß ausgebildeten Durchbrechungen oder Löchern 3E ist einer definierten Deagglomeration und Ausgabe, also Zerstäubung, der Formulierung 2 bzw. der Pulverpartikel der Formulierung 2 zuträglich.

Es ist anzumerken, dass die Abdeckung 3D mit den Löchern 3E aus einem beliebigen wirkstoffkompatiblen Material, je nach Anwendung insbesondere aus Plastik, Papier o. dgl., ggf. auch aus Metall bzw. Metallfolie, gebildet sein kann.

Die Abdeckung 3D kann auch als Einsatz gestaltet sein, beispielsweise durch ein in die Aufnahme 3 eingesetztes gitterartiges Element oder dergleichen, und/oder eine Baugruppe mit dem Abdeckelement 10A bilden. Besonders bevorzugt werden die Abdeckung 3D und das Abdeckelement 10A durch zwei übereinanderliegende Folien oder Schichten gebildet, insbesondere wobei die Abdeckung 3D die Lochstruktur enthält und das Abdeckelement 10A dem Feuchtigkeitsschutz der Formulierung 2 im ungeöffneten Zustand dient.

Die Abdichtung des Aufnahmeraums bzw. der Aufnahme 3A für die Formulierung 2 gegen Feuchtigkeit wird dann vorzugsweise von einer Deckfolie, hier insbesondere von dem Abdeckelement 10A, übernommen.

Alternativ oder zusätzlich kann jedoch auch der gesamte Inhalator 1 gegen Feuchtigkeit geschützt werden, beispielsweise durch eine nicht dargestellte äußere Umhüllung, Folienverpackung o. dgl.

Der dargestellte Inhalator 1 ist vorzugsweise als Einweginhalator zur einmaligen Verwendung ausgebildet. Jedoch kann der Inhalator 1 (unabhängig von der Ausführung der Abdeckung 3D bzw. unabhängig vom Öffnen durch Anstechen oder Abziehen) grundsätzlich auch zur mehrfachen Verwendung ausgebildet sein. Beispielsweise ist dann der Träger 3 wechselbar. Alternativ kann der Inhalator 1 ggfs. auch mehrere Träger 3 und/oder Aufnahmen 3A enthalten, die einzeln bzw. unabhängig voneinander offenbar sind, um dementsprechend einzeln oder unabhängig voneinander jeweils eine oder mehrere Dosen der Formulierung 2 ausgeben zu können. Einzelne Merkmale und Aspekte der verschiedenen Ausführungsformen können auch beliebig miteinander kombiniert, aber auch unabhängig realisiert oder bei sonstigen Konstruktionen von Inhalatoren eingesetzt werden.

Die vorliegende Erfindung ist nicht auf Inhalatoren beschränkt, sondern kann entsprechend auch bei sonstigen Zerstäubern eingesetzt werden. Dementsprechend ist der Begriff "Inhalator" vorzugsweise in einem weiteren Sinne auch dahingehend zu verstehen, dass er auch sonstige Spender oder Zerstäuber, insbesondere für medizinische oder sonstige therapeutische Zwecke, umfasst,

### Bezugszeichenliste

- 1: Inhalator
- 1A: Auslass
- 1B: Einlass
- 1C: Strömungsraum
- 1D: Öffnung
- 2: Formulierung
- 3: Träger
- 3A: Aufnahme
- 3B: Bodenelement
- 3C: Kante
- 3D: Abdeckung
- 3E: Loch
- 4: Federabschnitt
- 4A: Federsteg
- 4B: Schlitz
- 5: Flügel
- 6: Gehäuse
- 6A: Unterteil
- 6B: Oberteil
- 7: Öffnungseinrichtung
- 7A: Anstechelement
- 7B: Betätigungselement
- 7C: Federelement
- 8: Anschlag
- 9: Anschlag
- 10: Abdeckeinrichtung
- 10A: Abdeckelement
- 10B: Griffelement

- A: Achse
- L: Luftstrom
- P: Pulverstrom
- S: Schwingung

## Patentansprüche

1. Inhalator (1) zur Inhalation einer Formulierung (2) von einem vorzugsweise bandförmigen, streifenförmigen, blisterartigen und/oder folienartigen Träger (3), insbesondere Blister, wobei zum oder beim Austragen und/oder Dispergieren der Formulierung (2) der Träger (3) durch Anströmung von einen Luftstrom (L) in Schwingung (S) versetzbar ist,
wobei der Träger (3) nur einseitig und beweglich mittels eines zur Schwingung federelastisch verformbaren Federabschnitts (4) gehalten ist, und der Inhalator (1) derart ausgebildet ist, dass der Träger (3) mit einer definierten Bewegung Amplitude und/oder Frequenz in Schwingung (S) versetzbar ist, **dadurch gekennzeichnet, dass** der Federabschnitt (4) zwei parallel verlaufende Federstege (4A) und eine Durchbrechung aufweist.

2. Inhalator nach Anspruch 1, **dadurch gekennzeichnet, dass** der Federabschnitt (4) den Träger (3) quer zum Luftstrom (L) beweglich, aber in Richtung des Luftstroms (L) torsionssteif hält.

3. Inhalator nach Anspruch 1 oder 2, **dadurch gekennzeichnet, dass** der Träger (3) um eine im Bereich des Federabschnitts (4) liegende bzw. von dem Federabschnitt (4) gebildete Achse (A) schwenkbar bzw. bewegbar ist.

4. Inhalator nach einem der voranstehenden Ansprüche, **dadurch gekennzeichnet, dass** der Federabschnitt (4) blattartig ausgebildet ist.

5. Inhalator nach einem der voranstehenden Ansprüche, **dadurch gekennzeichnet, dass** der Träger (3) zumindest im Wesentlichen ausschließlich von einem freien bzw. dem Federabschnitt (4) entgegengesetzten Ende her vom Luftstrom (L) anströmbar ist, und/oder dass der Luftstrom (L) zumindest im Wesentlichen nur parallel zu einer Flachseite und/oder zumindest im Wesentlichen nur auf einer Flachseite des Trägers (3) am Träger (3) entlang strömt.

6. Inhalator nach einem der voranstehenden Ansprüche, **dadurch gekennzeichnet, dass** dem Träger (3) ein schrägstehender, vom Luftstrom (L) anströmbarer Flügel (5) zugeordnet ist, insbesondere wobei der Flügel (5) an einem freien Ende des Trägers (3) und/oder benachbart zu einem Lufteinlass (1B) des Inhalators (1) angeordnet ist.

7. Inhalator nach einem der voranstehenden Ansprüche, **dadurch gekennzeichnet, dass** der Inhalator (1) eine Öffnungseinrichtung (7) zum Öffnen des Trägers (3) bzw. eines Aufnahmeraums (3A) mit der Formulierung (2), besonders bevorzugt durch Anstechen, aufweist.

8. Inhalator nach Anspruch 7, **dadurch gekennzeichnet, dass** die Öffnungseinrichtung (7) von einer vorzugsweise manuell verformbaren Gehäusewandung gebildet ist und/oder mindestens ein federnd und/oder rückstellend gelagertes Anstechelement (7A) aufweist.

9. Inhalator nach einem der voranstehenden Ansprüche, **dadurch gekennzeichnet, dass** der Inhalator (1) mindestens einen Anschlag (8, 9) zur Begrenzung der Schwingung (S) des Trägers (3) aufweist, insbesondere wobei der mindestens eine Anschlag (8, 9) von einem Gehäuse (6) des Inhalators (1) gebildet ist.

10. Inhalator nach einem der voranstehenden Ansprüche, **dadurch gekennzeichnet, dass** der Träger (3) oder eine Abdeckung (3D) des Trägers (3) zumindest partiell durch eine Abdeckeinrichtung (10) abgedeckt ist, die zum Öffnen des Trägers (3) bzw. der Abdeckung (3D) für die anschließende Abgabe der Formulierung (2) manuell öffenbar oder entfernbar ist.

11. Inhalator nach Anspruch 10, **dadurch gekennzeichnet, dass** die Abdeckeinrichtung (10) ein umgelegtes Abdeckelement (10A) und vorzugsweise ein zugeordnetes Griffelement (10B) zum Abziehen des Abdeckelements (10A) von der Abdeckung (3D) aufweist, insbesondere wobei das Abdeckelement (10A) und/oder Griffelement (10B) aus dem Inhalator (1), insbesondere einem Mundstück oder Auslass (1A) des Inhalators (1), herausragt.

12. Inhalator nach Anspruch 10 oder 11, **dadurch gekennzeichnet, dass** die Abdeckung (3D) mindestens eine Durchbrechung, insbesondere mehrere Löcher (3E), aufweist, die von der Abdeckeinrichtung (10) bzw. einem Abdeckelement (10A) der Abdeckeinrichtung (10) bei geschlossenen Träger (3) abgedeckt ist bzw. sind.

13. Inhalator nach einem der voranstehenden Ansprüche, **dadurch gekennzeichnet, dass** der Inhalator (1) als Einweginhalator mit darin angeordnetem Träger (3) ausgebildet ist.

14. Inhalator nach einem der voranstehenden Ansprüche, **dadurch gekennzeichnet, dass** die Durchbrechung ein Schlitz oder Längsschlitz (4B) ist.

## Claims

1. Inhaler (1) for inhalation of a formulation (2) from a preferably band-shaped, strip-shaped, blister-like and/or film-like carrier (3), in particular blister, wherein for or during delivery and/or dispersion of a formulation (2) the carrier (3) can be set into oscillation (S) by impact of an airflow (L),
wherein the carrier (3) is held only on one side and moveably by means of a spring portion (4) that is spring-resiliently deformable for oscillation,
and the inhaler (1) is embodied such that the carrier (3) can be set into oscillation (S) with a defined movement, amplitude and/or frequency,
**characterized in**
**that** the spring portion (4) comprises two spring bars (4A) extending parallel to one another and a through hole.

2. Inhaler according to claim 1, **characterized in that** the spring portion (4) holds the carrier (3) so that it is moveable transversely to the airflow (L) but torsionally rigid in the direction of the airflow (L).

3. Inhaler according to claim 1 or 2, **characterized in that** the carrier (3) is pivotable and/or moveable about an axis (A) located in the region of the spring portion (4) and/or formed by the spring portion (4).

4. Inhaler according to one of the preceding claims, **characterized in that** the spring portion (4) is configured leaf-like.

5. Inhaler according to one of the preceding claims, **characterized in that** the carrier (3) can be impacted by the airflow (L) at least essentially only from a free end or the end opposite the spring portion (4), and/or that the airflow (L) flows along the carrier (3) at least essentially only parallel to a flat side and/or at least essentially only on a flat side of the carrier (3).

6. Inhaler according to one of the preceding claims, **characterized in that** an oblique fin (5) which can be impacted by the airflow (L) is assigned to the carrier (3), in particular wherein the fin (5) is arranged at a free end of the carrier (3) and/or adjacent to an air inlet (1B) of the inhaler (1).

7. Inhaler according to one of the preceding claims, **characterized in that** the inhaler (1) comprises an opening device (7) for opening the carrier (3) and/or a holding chamber (3A) with the formulation (2), particularly preferably by piercing.

8. Inhaler according to claim 7, **characterized in that** the opening device (7) is formed by a preferably manually deformable housing wall and/or comprises at least one resiliently and/or resettingly mounted piercing element (7A).

9. Inhaler according to one of the preceding claims, **characterized in that** the inhaler (1) comprises at least one stop (8, 9) for limiting the oscillation (S) of the carrier (3), in particular wherein the at least one stop (8, 9) is formed by a housing (6) of the inhaler (1).

10. Inhaler according to one of the preceding claims, **characterized in that** the carrier (3) or a cover (3D) of the carrier (3) is at least partially covered by a covering device (10) which can be manually opened or removed in order to open the carrier (3) and/or the cover (3D) for the subsequent dispensing of the formulation (2).

11. Inhaler according to claim 10, **characterized in that** the covering device (10) comprises a folded-back cover element (10A) and preferably an associated grip element (10B) for pulling the cover element (10A) off the cover (3D), in particular wherein the cover element (10A) and/or grip element (10B) projects from the inhaler (1), in particular a mouthpiece or outlet (1A) of the inhaler (1).

12. Inhaler according to claim 10 or 11, **characterized in that** the cover (3D) comprises at least one through hole, in particular several holes (3E), which is or are covered by the covering device (10) and/or a covering element (10E) of the covering device (10) when the carrier (3) is closed.

13. Inhaler according to one of the preceding claims, **characterized in that** the inhaler (1) is embodied as a single-use inhaler with a carrier (3) arranged therein.

14. Inhaler according to one of the preceding claims, **characterized in that** the through hole is a slit or longitudinal slot (4B).

## Revendications

1. Inhalateur (1) pour l'inhalation d'une formulation (2) à partir d'un support (3) de préférence en forme de bande, en forme de ruban, du type emballage coque et/ou du type feuille, en particulier d'un emballage-coque, dans lequel le support (3), pour ou pendant la distribution et/ou la dispersion de la formulation (2), peut être mis en vibration (S) en étant exposé à un flux d'air (L),
dans lequel le support (3) n'est retenu que par un côté et de façon mobile au moyen d'une partie ressort (4) déformable élastiquement aux fins de vibration,
et l'inhalateur (1) est conçu de telle manière que le support (3) peut être mis en vibration (S) avec une amplitude de mouvement définie et/ou à une fréquence de mouvement définie,
**caractérisé en ce**
**que** la partie ressort (4) comprend deux éléments de liaison de ressort (4A) s'étendant parallèlement et une ouverture.

2. Inhalateur selon la revendication 1, **caractérisé en ce que** la partie ressort (4) porte le support (3) de manière à ce qu'il soit mobile transversalement au flux d'air (L) mais à ce qu'il soit rigide en torsion en direction du flux d'air (L).

3. Inhalateur selon la revendication 1 ou 2, **caractérisé en ce que** le support (3) peut pivoter ou se déplacer autour d'un axe (A) situé dans la zone de la partie ressort (4) ou formé par la partie ressort (4).

4. Inhalateur selon l'une quelconque des revendications précédentes, **caractérisé en ce que** la partie ressort (4) est du type lame.

5. Inhalateur selon l'une quelconque des revendications précédentes, **caractérisé en ce que** le support (3) peut être exposé au flux d'air (L), au moins sensiblement exclusivement depuis une extrémité libre ou opposée à la partie ressort (4), et/ou **en ce que** le flux d'air (L) s'écoule au moins sensiblement uniquement parallèlement à une face plate et/ou au moins sensiblement uniquement sur une face plate du support (3) le long du support (3).

6. Inhalateur selon l'une quelconque des revendications précédentes, **caractérisé en ce qu'**une aile (5) inclinée, pouvant être exposée au flux d'air (L), est associée au support (3), dans lequel l'aile (5) étant notamment agencée à une extrémité libre du support (3) et/ou au voisinage d'une entrée d'air (1B) de l'inhalateur (1).

7. Inhalateur selon l'une quelconque des revendications précédentes, **caractérisé en ce que** l'inhalateur (1) comprend un dispositif d'ouverture (7) destiné à ouvrir le support (3) ou un espace de réception (3A) contenant la formulation (2), de manière particulièrement préférée par perçage.

8. Inhalateur selon la revendication 7, **caractérisé en ce que** le dispositif d'ouverture (7) est formé par une paroi de boîtier de préférence déformable manuellement et/ou comprend au moins un élément de perçage (7A) monté élastique et/ou à rappel.

9. Inhalateur selon l'une quelconque des revendications précédentes, **caractérisé en ce que** l'inhalateur (1) comprend au moins une butée (8, 9) destinée à limiter la vibration (S) du support (3), dans lequel ladite au moins une butée (8, 9) est en particulier formée par un boîtier (6) de l'inhalateur (1).

10. Inhalateur selon l'une quelconque des revendications précédentes, **caractérisé en ce que** le support (3) ou un cache (3D) du support (3) est recouvert au moins partiellement par un dispositif de recouvrement (10), qui peut être ouvert ou retiré manuellement afin d'ouvrir le support (3) ou le cache (3D) pour distribuer ensuite la formulation (2).

11. Inhalateur selon la revendication 10, **caractérisé en ce que** le dispositif de recouvrement (10) comprend un élément de recouvrement (10A) rabattu et de préférence un élément de préhension (10B) associé destiné à enlever l'élément de recouvrement (10A) du cache (3D), dans lequel l'élément de recouvrement (10A) et/ou l'élément de préhension (10B) fait notamment saillie de l'inhalateur (1), en particulier d'un embout buccal ou d'un orifice de sortie (1A) de l'inhalateur (1).

12. Inhalateur selon la revendication 10 ou 11, **caractérisé en ce que** le cache (3D) présente au moins une ouverture, notamment plusieurs trous (3E), qui est recouverte ou sont recouverts par le dispositif de recouvrement (10) ou un élément de recouvrement (10A) du dispositif de recouvrement (10) lorsque le support (3) est fermé.

13. Inhalateur selon l'une quelconque des revendications précédentes, **caractérisé en ce que** l'inhalateur (1) est réalisé sous la forme d'un inhalateur à usage unique dans lequel est agencé le support (3).

14. Inhalateur selon l'une quelconque des revendications précédentes, **caractérisé en ce que** l'ouverture est une fente ou une fente longitudinale (4B).
